# DEMANDE DE BREVET EUROPEEN

(11) **EP 1 582 214 A2**
(43) Date de publication de la demande: **05.10.2005**
(21) Numéro de dépôt: 05290624.5
(22) Date de dépôt: 22.03.2005
(51) Int. Cl.: A61K 31/737, A61K 35/12, A61P 19/02

(54) **Utilisation des chondroitine mono- et disulfates en thérapeutique**

(30) Priorité: 24.03.2004 FR 0403020
(71) Demandeur: Laboratoire Genevrier, 06600 Sophia-Antipolis (FR)
(72) Inventeur: Vacher, Dominique, 06250 Mougins (FR)
(74) Mandataire: Burtin, Jean-François

(57) **Abrégé**

La présente invention se rapporte au domaine des nécessités de la vie et plus particulièrement au domaine de la chimie biologique.

Elle a précisément pour objet l'utilisation d'acides chondroïtine 2, 4 et/ou 2,6 disulfuriques en vue de la réalisation d'un médicament destiné à assurer la conservation ou la régénération des cartilages.

Un tel médicament est destiné à l'administration par voie orale ou parentérale à des doses s'échelonnant de 0,2 g à 2 g par prise unitaire.

## Description

La présente invention s'adresse au domaine des nécessités de la vie, et plus particulièrement au domaine de la chimie biologique.

Elle a plus particulièrement pour objet l'utilisation en thérapeutique de composés dérivés de chondroïtines sulfuriques, extraits de cartilages d'origines diverses.

Elle a spécifiquement pour objet l'utilisation en thérapeutique des chondroïtines constituées essentiellement, mais non exclusivement, de disaccharide sulfatés en position 2, 4, 6 et/ou de disaccharides di-sulfatés en position 2,6; et pouvant inclure des disaccharides non sulfatés, en tant que facteurs de conservation et de régénération des cartilages.

Ces chondroïtines sont obtenues par clivage enzymatique des protéinoglycanes déjà sulfatées, contenus dans les cartilages de différentes espèces animales, et notamment d'origine bovine, porcine ou ichtyque. Dans ces cartilages, les chondroïtines sulfates se présentent sous forme de polysaccharides à longue chaîne où ils sont associés à une protéine axiale, elle-même liée à des keratanes sulfates, et l'ensemble est connu sous le nom d'aggrecane.

Le clivage enzymatique, notamment sous l'action de chondroïtinase ABC, a pour effet de fournir un mélange de disaccharides que l'on appelle :
- d'acide chondroïtine 6-sulfurique,
- d'acide chondroïtine 4-sulfurique,
- d'acide chondroïtine disulfurique, présentant un sulfate en 2 de l'acide glucuronique et un sulfate en 6 de la N-acétyl galactosamine.
   Selon l'origine du cartilage, les pourcentages en ces différents disaccharides varient. Les tissus cartilagineux d'origine ichtyque fournissent des pourcentages plus élevés en acide 2,6-chondroïtine disulfurique et en acide chondroïtine 6-sulfurique. Au contraire, les trachées d'origine porcine, fournissent des chondroïtines plus riches en acide chondroïtine 4-sulfurique (environ 60 %). Les tissus cartilagineux d'origine bovine fournissent un mélange d'acides chondroïtine 4-sulfurique et 6-sulfurique. Le mélange est souvent accompagné de la présence de molécules non sulfatées.
   Selon N. Volpi Current Drug Target (2004) 4, 119-127, les chondroïtines sulfates constituent des polyanions et certaines de leurs propriétés sont liées à leur forte charge qui est capable d'attirer l'eau dans les tissus et de les hydrater. Les chaînes d'acides chondroïtine sulfuriques sont synthétisées par des cellules liées d'une manière covalente aux protéines qui sont sécrétées dans la matrice extracellulaire, sous forme de protéoglycanes. Plusieurs familles de protéoglycanes, portant des chaînes d'acides chondroïtine sulfuriques, ont été caractérisées, y compris deux des familles les plus importantes de protéoglycanes trouvées dans la matrice extracellulaire des tissus connectifs : la famille des aggrécans et la famille des protéoglycanes riches en motifs répétitifs leucine.
- La famille des aggrécans est constituée de fractions de haut poids moléculaire (>500 kDa) qui s'agrègent à l'extérieur de la cellule en se liant à l'acide hyaluronique. Elles jouent un rôle important dans les propriétés physiques et chimiques du cartilage.

Sans vouloir être lié par des considérations d'ordre théorique, il apparaît que, dans les molécules d'aggrecan et autres protéoglycanes, les acides chondroïtine sulfuriques existent sous forme de longues chaînes dont le motif est l'acide D-glucuronique liée par une liaison (1→3) à un N-acétylgalactosamine. Plusieurs types de sulfatation sont observées : pour la N-acétylgalactosamine les sulfatations se retrouvent préférentiellement en 4 et/ou en 6 et en position 2 pour l'acide D-glucuronique.

Le clivage enzymatique peut être effectué par une chondroïtinase et notamment par une chondroïtinase C ou par une chondroïtinase ABC qui fournit notamment les disaccharide 6-sulfurique, 4-sulfurique et 2,6-disulfurique, accompagnés de produits plus sulfatés. Des disaccharides non sulfatés présents dans toutes les origines sont également libérés par cette digestion enzymatique.

L'analyse des chondroïtines sulfates peut être effectuée par RMN et montre des spectres différents qui sont la conséquence de la présence des différentes sulfatations et de leur proportion respective. De même, l'analyse par IRTF met en évidence dans la région située entre 1000 et 500 cm⁻¹, et plus particulièrement entre 950 - 700 cm⁻¹, des pics caractéristiques de chacun des acides chondroïtine sulfuriques. Les différentes chondroïtines d'origine porcine, ichtyque (requin) et bovine possèdent des spectres différents qui attestent de la présence et des proportions des différents disaccharides sulfuriques en quantités différentes.

En outre, une réaction de β-élimination (entre le xylose et la sérine), réalisée sur les chondroïtines sulfates après extraction, permet d'évaluer le taux et la masse moléculaire des protéines qui resteraient liées malgré les processus de purification. La technique d'analyse utilisée ici est la chromatographie liquide sur colonne d'exclusion avec comme détecteur mais non exclusivement, un détecteur à indice de réfraction. Cette méthode renseigne sur la présence de protéines résiduelles liées.

La quantité de dérivés 6-sulfuriques obtenue est dépendante comme pour les dérivés 4 sulfurique, de l'origine animale et du tissu d'extraction. Ainsi pour toutes les origines, mis à part le requin les valeurs sont toujours inférieures à celles du dérivé 4-sulfurique. Pour le requin, les dérivés sulfuriques représentent au minimum 40 % de la totalité des disaccharides. L'acide chondroïtine 2,6-disulfurique ne se trouve en pratique à des concentrations supérieures à 10 % que dans les mélanges d'origine ichtyque.

Les acides chondroïtine sulfuriques ou disulfuriques résultant du clivage enzymatique des tissus cartilagineux jouent un rôle important dans les propriétés physiques et chimiques du cartilage. A concentrations élevées ils attirent l'eau dans les tissus de telle sorte que le collagène est placé sous tension, ce qui à son tour, contribue à la résistance mécanique du cartilage aux forces de compression. Ces molécules synthétisées par les chondrocytes et secrétées dans l'espace intercellulaire, sont liées aux hyaluronans et forment des complexes moléculaires de haut poids moléculaire.

Dans les conditions physiologiques les chondrocytes articulaires sont responsables de l'évolution lente de la matrice extra cellulaire ce qui constitue une des causes des lésions des tissus.

Au cours du vieillissement, la taille de ces agrégats moléculaires, la teneur en acides chondroïtine sulfuriques, et la capacité de liaison des aggrécans tend à diminuer. Ces modifications peuvent être considérées comme un des facteurs qui interviennent dans la diminution de la capacité du cartilage à se réparer en cas de lésion et entraînent une prédisposition à des lésions ostéoarthritiques. Une fois le processus arthritique mis en place, les interleukines et notamment l'interleukine IL-1, jouent un rôle très important dans le métabolisme des aggrécans, en diminuant les processus de synthèse et en augmentant leur dégradation. La perte d'aggrécans conduit le réseau de collagène à une destruction mécanique et contribue à la progression de l'arthrose.

C'est pourquoi, les thérapeutiques qui manifestent des effets positifs "in vitro" sur l'anabolisme des aggrécans et/ou sur les capacités à empêcher les effets négatifs d'IL-1 sur le catabolisme des aggrécans sont susceptibles de retarder ou d'inhiber le processus de développement de la maladie ostéo-arthritique.

Les polysaccharides polysulfatés, et notamment ceux constitués de disaccharides disulfatés ont été utilisés chez des malades atteints d'osteo-arthrose et ont montré des résultats thérapeutiques importants, en particulier en ralentissant la progression de la maladie. Les disaccharides mono et/ou disulfatés peuvent être administrés par voie orale et peuvent atteindre des concentrations suffisantes dans les liquides biologiques - comme le liquide synovial- et dans les articulations pour exercer des effets bénéfiques.

L'utilisation de molécules marquées au [³⁵S] a montré des concentrations sériques importantes, et en particulier chez l'animal où l'on constate un tropisme sélectif vis-à-vis des tissus riches en GAG, tels que les disques intervertébraux, les yeux et le cartilage des articulations. D'une manière semblable, les acides chondroïtine mono- ou disulfatés manifestent un tropisme pour l'articulation du genou, ainsi qu'il a été mis en évidence par scanning (balayage) de l'os par CS marqué au 99 Tc.

Les acides chondroïtine mono- ou disulfuriques manifestent un effet protecteur sur l'endommagement du cartilage. Des études de dégradation du cartilage sur un modèle de lapin, induite par injection intra articulaire de chymopapaïne, ont montré un effet préventif sur la dégradation des cartilages. Les acides chondroïtine mono- ou disulfuriques manifestent "in vitro" des augmentations dans le taux de synthèses des aggrécans sur des chondrocytes humains et par là, contribuent à augmenter la teneur en protéines de la matrice, principalement les PG, dans l'environnement immédiat des chondrocytes, et de ce fait empêchent leur dédifférenciation.

Les études pharmacologiques effectuées avec les chondroïtines formées de disaccharides 2,4 ou 6-sulfuriques ainsi que de 2,6-disulfurique montrent une augmentation de la production et de la capacité d'aggrégation des protéoglycanes (PG) nouvellement synthétisés qui s'accumulent tout autour des chondrocytes mis en incubation dans des conditions de base. En outre, les acides chondroïtine mono- ou disulfuriques ont pour effet d'inverser au moins partiellement la désagrégation induite par IL-1 des protéoglycanes, et cet effet est observé "in vitro", même en l'absence de toute intervention biologique sur les cellules. Cet effet ne modifie pas le phénotype de chondrocyte comme il est mis en évidence par une expression stable de collagène de type II et d'aggrécan, sans hyper régulation du collagène de type I.

Cet effet n'est pas lié à la sulfatation de la molécule car l'héparine est inactive pour augmenter la synthèse des protéoglycanes.

De plus, les acides chondroïtine mono- et disulfatés augmentent la proportion de PG nouvellement synthétisés qui s'accumulent dans l'espace intercellulaire. Cette présence de PG en quantités importantes dans l'environnement des chondrocytes est un élément clé pour assurer la conservation du phénotype des chondrocytes et les propriétés physico-mécaniques du cartilage.

Les effets bénéfiques des acides chondroïtine mono- ou disulfuriques peuvent trouver une explication dans une augmentation de la synthèse de filaments plus longs d'hyaluronane, dans la quantité d'acide hyaluronique synthétisée et dans l'inhibition des activités enzymatiques, comme celle de l'élastase et de l'agrécanase ou de la cathepsine B. On peut donc supposer que les acides chondroïtine mono- ou disulfuriques peuvent avoir un effet sur la régulation des actions des métalloprotéases induites par IL-1 β et préservent ainsi le stock de PG non dégradés.

Il apparaît principalement que les acides chondroïtine mono- et disulfuriques se distinguent par des interactions physico-chimiques avec les PG monomères. Un effet d'aggrégation similaire a été observé avec les acides chondroïtine sulfuriques lorsqu'ils sont mélangés avec des PG marqués au [³⁵S], désagrégés juste avant d'être analysés par chromatographie. Cet effet est observé avec tous les types d'acide chondroïtine sulfurique, quelle que soit leur origine mais à des degrés divers. Par exemple, l'acide chondroïtine sulfurique extrait de cartilages de porc manifeste un pouvoir aggrégant puissant avec une augmentation des aggrégats de haut poids moléculaire (AHMW) de plus de 80 % manifesté par la mesure de radioactivité totale, alors que les acides extraits de cartilage d'autres espèces animales, telles que, le poulet ou le requin ont donné des augmentations de réaggrégation de 70 % en présence d'interleukine IL-1β par rapport à l'état normal.

Les acides chondroïtine mono- ou disulfuriques de l'invention augmentent le pourcentage de macromolécules sulfatées fonctionnelles dans l'environnement direct des chondrocytes "in vitro", principalement en manifestant des propriétés d'aggrégation des PG. Dans le cas où les cartilages sont endommagés, la rétention péri-cellulaire d'aggrécans volumineux, après induction par les acides chondroïtine mono- ou disulfuriques de l'invention, joue un rôle favorable pour retarder, ou mieux supprimer, le processus de dédifférenciation et pour amener une récupération d'un phénotype différencié.

Les acides chondroïtine mono (2,4 ou 6) sulfatés et l'acide chondroïtine 2,6-disulfurique de l'invention sont utilisés à des fins thérapeutiques, notamment par voie orale ou parentérale, sous forme de compositions pharmaceutiques telles que comprimés, dragées, gélules, pilules, cachets, poudres aromatisées ou non, ou d'injections destinées à la voie intramusculaire et intra-articulaire, sous forme d'ampoules, de fioles monodoses ou de seringue auto-injectables.

Les doses utilisées peuvent varier considérablement en fonction de l'ancienneté ou de l'évolution de la maladie. Les acides chondroïtine sulfuriques selon l'invention sont pratiquement dépourvus de toxicité et trouvent de ce fait un emploi très large en thérapeutique. Une dose usuelle pour la voie orale ou parentérale s'échelonne de 0,2 g à 2 g par prise unitaire et de préférence de 0,4 g à 0,6 g par prise.

### EXEMPLE I - Obtention des chondroïtine sulfates par hydrolyse enzymatique

Le tissu cartilagineux, en l'espèce les morceaux de trachée de bovins ou de porcins, est haché en petits fragments et soumis à une hydrolyse enzymatique en utilisant comme enzyme protéolytique l'enzyme commercialisé sous la dénomination Prolyve 100® par la société LYVEN. Il s'agit d'une protéase alcaline liquide, obtenue à partir d'une souche sélectionnée de Bacillus licheniformis. La fermentation est orientée pour produire essentiellement une protéase alcaline de type sérine, appelée aussi « subtilysine Carlsberg » (EC 3.4.21.14).

Les chondroïtines sont séparées après filtration par addition d'éthanol sous agitation. Le précipité est essoré puis lavé par un mélange eau/ethanol. Le précipité est repris par un mélange eau/acétate de sodium, puis re-précipité par de l'éthanol sous agitation puis essoré, séché en étuve, broyé et tamisé sur maille de 450 µm.

La chondroïtine ainsi obtenue est analysée en RMN du proton, ce qui permet de caractériser deux pics très aigus à 2,04 ppm (6S) et à 2,02 ppm (4S).

Les mêmes pics se retrouvent pour les chondroïtines d'origine ichtyque, bovine (essentiellement du 4 S) et porcine (pics à 2,02 et à 2,04 ppm). La chondroïtine d'origine ichtyque est la seule à fournir un pic important, correspondant à l'acide 6-chondroïtine sulfurique (voir figure 1).

L'IRTF en pastilles de KBr montre entre 1 500 et 1 000 cm₋₁ les pics caractéristiques des chondroïtines de différentes origines (voir figure 2).

### EXEMPLE II - Etude pharmacologique des acides chondroïtine sulfuriques

### 1. Action des acides chondroïtine sulfuriques sur les aggrécans et sur l'expression de l'ARNm du collagène de type II dans les chondrocytes.

Des chondrocytes articulaires ont été mis en culture à une densité élevée pendant 6 jours. Les cellules sont incubées en présence ou en l'absence d'effecteurs à 10 ou 100 µg/ml en présence ou en l'absence d'IL-1β pendant 20 heures. Un transcript unique d'ARN de 8,9 kb et un transcript unique de procollagène II α 1 de 5 kb ont été observés dans des conditions de base.

L'addition de 10 ou de 100 µg/ml d'acide chondroïtine sulfurique commercial (CS) n'a pas d'effet sur le rapport des ARNₘ aggrécan/GAPDH ou sur le rapport procollagène II α 1/GAPDH en comparaison avec des cellules non traitées.

En outre, aucune expression des produits de transcription (transcripts) de procollagène I 4,8 et 5,8 α 1 n'a été observée pendant que les fibroblastes cutanés étaient utilisés en tant que témoins positifs.

Le traitement des cellules avec IL-1 β a entraîné une diminution faible mais significative des taux d'aggrécan et des taux d'ARNₘ de procollagène II α 1. L'ajout de CS, n'a pas eu d'effet. Aucune expression d' ARNₘ de procollagène 1 n'a été détectable.
En résumé, le mode d'action des CS ne se situe vraisemblablement pas au niveau de la modulation de la synthèse des ARNₘ du Col II ou des aggrécans.

### 2. Modulation de la production des protéoglycanes nouvellement synthétisés (PG) par CS.

L'action des CS sur la synthèse de PG a été étudiée par incorporation de (³⁵SO₄] dans des cultures de chondrocytes traitées par le CS. Des chondrocytes ont été mis en culture comme décrit ci-dessus. L'incubation avec ou sans effecteur a été réalisée dans du milieu DMEM (DULBECCO Modified Eagle Médium) dépourvu de sulfate auquel on a ajouté du sulfate marqué radioactivement. Dans les conditions de base, sans aucun effecteur, le niveau de PG sulfate (³⁵SO₄] total, extrait du milieu de culture (M), et du pool de cellules correspondantes (C) a été en moyenne (± SEM) de 3,42 dpm x 10⁻⁴ /10 µg d'ADN avec 36 % récupéré dans la couche de cellules. L'addition de CS n'a pas modifié sensiblement la quantité de PG sulfate [³⁵SO₄] secrété dans le milieu de culture alors qu'une augmentation importante a été observée dans le pool cellulaire. Le taux de PG sulfate [³⁵SO₄] mesuré dans le pool cellulaire a été augmenté de 68 % au-dessus des valeurs témoins (p < 0,01) en présence de 10 µg/ml de CS représentant ainsi 45 % du PG sulfate [³⁵SO₄] total. Aucun effet de stimulation supplémentaire n'a été observé lorsqu'on a ajouté CS à des doses plus élevées.

En présence de concentrations croissantes (2,10 ou 20 ng/ml) d'IL-1β seul, le PG sulfate [³⁵SO₄] total récupéré dans le pool cellulaire a été notablement diminué, d'une manière proportionnelle aux doses, représentant ainsi 37%, 10% et 6% de PG sulfate [³⁵SO_{4]}. néo-synthétisé (Tableau 3). L'addition de CS à 10 ou 100 µg/ml avec 20 ng d'IL-1β n'a pas modifié la quantité de PG sulfate [³⁵SO_{4]} secrété dans le milieu de culture alors qu'une importante stimulation de PG [³⁵SO_{4]} sulfaté récupéré dans le pool cellulaire a été observée.
CS a donc une action sur la synthèse des PG synthétisés par les chondrocytes.

### EXEMPLE III - Modulation des propriété physiques de PG néosynthétisé par CS

L'action des CS sur l'aggrégation des PG néosynthétisés a été investiguée. L'IL 1β a pour propriété de désagréger les PG ce qui se traduit lorsque l'on réalise une séparation par chromatographie d'exclusion des PG synthétisés par les chondrocytes, par une augmentation de complexes de plus bas poids moléculaires. Dans les conditions de base, plus de 80 % (± 5%) du PG sulfate [³⁵SO₄] ont été éluées sous forme de complexes agrégés de haut poids moléculaire (AHMW) (Kₐᵥ = 0,10 - 0,40, zone en pointillé) avec moins de 10 % (± 3%) sous forme de PG non agrégé (Kaᵥ 0,40 à Kaᵥ 0,60).

Dans les cellules traitées par IL-1β, une quantité décroissante d'une manière dose-dépendante, de PG aggrégée par (AHMW) a été observée en comparaison aux conditions témoin (figure 2B) représentant ainsi 73 (± 5%), 59 (± 6 %), et 38(± 5%) de la radioactivité totale sur la colonne avec 2, 10, et 20 ng/ml de IL-1β respectivement. La quantité de complexes agrégés AHMW récupérés dans les échantillons de PG sulfates [³⁵SO₄] à partir de cellules traitées par 20 ng/ml d'IL-1β et soit 10 ou 100 µg/ml de CS a été respectivement augmenté jusqu'à 60 % (± 4%) et Υ (± 5%) (p < 0,01) en comparaison avec 38 % (± 6%) en présence de IL- 1β seul.

Ces résultats sont la moyenne (±SD) de trois expériences séparées effectuées avec des chondrocytes provenant d'animaux différents.

Les CS sont donc capables de limiter la désagrégation de complexes de PG de haut poids moléculaire.

### EXEMPLE IV - Effets de CS sur l'expression de l'ARNₘ de MMP induite par 1L-1β et l'activité gélatinolytique

L'action des CS sur l'expression des MMP, protéases naturellement synthétisées par les chondrocytes, a été étudiées par "Northern Blot" après extraction des ARN de chondrocytes traités.

L'expression d'ARNm de MMP - 1, -3 et - 1,3 a été augmentée fortement quand les cellules ont été traitées par IL-1β alors qu'aucun signal détectable n'a été observé dans les conditions témoin (DMEM dépourvu de sérum) ou en présence de CS (10 µg/ml) seul.

L'addition simultanée de IL-1β et de CS aux cellules de la culture n'a pas modifié les taux d'ARNm des MMP.

L'exposition des cellules à IL-1β plus CS (10 µg/ml) n'a pas modifié l'activité gélatinolytique principale observée dans les conditions basales d'un poids moléculaire apparent à 72 kDa (MMP-2), ni les activités supplémentaires observées en présence de IL-1β à un poids moléculaire apparent de 92 kDa et de 53/58 kDa, correspondant vraisemblablement à MMP-1,3 ou 9 et à MMP-1 ou 3 respectivement.

L'action des CS ne se situe pas au niveau de l'expression des MMPs investiguées dans ces expériences, ni au niveau de l'activité gélatinolytique dans les conditions expérimentales.

### EXEMPLE V : Effet de l'addition de CS ou d'autres échantillons de chondroïtine sulfate « in vitro » avec des PG sulfates [³⁵SO₄] dégradés induits par IL-1 β juste avant d'être appliqués sur la colonne de Sépharose.

L'action de différentes sources de CS sur l'agrégation physique des PG synthétisés par les chondrocytes a ici été investiguée directement en mettant en contact les PG avec les CS. Des échantillons de quantités semblables de PG sulfates [³⁵SO₄] provenant de chondrocytes traités par 20 ng/ml d'IL-1β ont été mélangés avec des CS de différentes origines avant d'être appliqués sur la colonne de Sépharose.

Pour chaque profil d'élution, la quantité de PG agrégé éluée entre Kav 0,10 - 0,40 a été quantifiée et exprimée en tant que pourcentage de la radioactivité totale éluée sur la colonne.

Les résultats figurent dans le tableau 3.

Alors qu'en présence d'IL-1β (20 ng/ml) seul, 49 ± 6 % du PG [³⁵S] sulfaté ont été élués sous forme de PG agrégés par AHMW (Kₐᵥ 0,1 - 0,4), l'addition de chaque polymère a augmenté notablement la proportion de molécules agrégées AHMW jusqu'à plus de 70 % de la radioactivité totale. Le pouvoir d'aggrégation maximale (plus de 80 % de la radioactivité totale) a été observé avec des CS d'origine porcine (CS 204, CS-206 et CS-IG 336) alors que ceux d'autres origines étaient moins actives. Très clairement, selon la source de CS, une action de réagrégation plus ou moins prononcée a été mise en évidence dans ces conditions expérimentales.

### EXEMPLE VI - Exemple de formulation galénique

Acide chondroïtine dsulfurique d'origine porcine contenant environ 60 % d'acide chondroïtine, 4-sulfurique 500 g,
Lactose 50 g,
Stéarate de Magnésium 20 g
pour 1.000 gélules d'un poids moyen de 0,55 g
Colorant jaune qs

**Tableau I**

| Effecteur (mg/ml) | Incorporation de [³⁵SO₄] sulfate dans les protéoglycanes (dpm x 10⁻⁴ / 10 mg d'ADN) | | |
|---|---|---|---|
| | CS | | |
| | Médium (M) | Cellules (C) | C / M+C (%) |
| Aucun | 2,19 ± 0,12 | 1,23 ± 0,05 | 36 |
| 10 | 2,53 ±0,19 | 2,07 ± 1,10** | 45 |
| 100 | 2,20 ±0,13 | 2,19 ±0,12** | 50 |
| 200 | 2,31 ± 0,13 | 1,97 + 1,12** | 46 |
| 500 | 2,61 ± 0.17** | 2,05 ± 0,1 | 44 |

| | | | |
|---|---|---|---|
| ** p<0,01 valeurs dans les cellules traitées par IL-1β par rapport aux témoins | | | |

Effet de CS sur l'incorporation totale de [³⁵SO₄] sulfate dans les protéoglycanes néosynthétisés

**Tableau 2 :**

| IL-1 β (ng/ml) | Incorporation de [³⁵SO₄] dans les protéoglycanes (dpm x 10⁻⁴/10 mg d'ADN) | | |
|---|---|---|---|
| | MEDIUM (M) | Couche de cellules (C) | C/M+C (%) |
| | 3,12 ± 022 | 1,76 ± 0,12 | 36 |
| 2 | 3,40 ± 0,21 | 2,01 + 0,13 | 37 |
| 10 | 3,42 ± 0,17 | 0,38 ± 0,05*** | 10 |
| 20 | 3,58 ± 0,19 | 0,24 ± 0,007*** | 6 |
| IL1 20 + CS 10 | 3,46 ± 0,29 | 1,31 ± 0,18 | 27 |
| IL1 20 + CS 100 | 3,26 ± 0,28 | 1,60 ± 0,10 | 33 |

| | | | |
|---|---|---|---|
| *** p<0,001 valeurs sur des cellules traitées par IL-1 β en comparaison avec des valeurs témoins sans effecteur | | | |

### Effet de IL-1 β sur l'incorporation de [³⁵SO₄] dans les macromolécules précipitables par CPC néosynthétisées par les chondrocytes. Les données ±SEM sont la moyenne de trois expériences effectuées sur quatre puits traités d'une manière similaire.

**Tableau 3 :**

| Effecteur | Origine | PM | Protéine (%) | Chondroitine sulfate de sodium (%) | Rapport SO3/C00H | HMW Proteoglycanes (Kₐᵥ 0.10-0.40) (%) |
|---|---|---|---|---|---|---|
| 10ng/ml IL-1β (n=8) | | | | | | 49±6 |
| CS 1 (n=8) | Porcine | 22 302 | 1,6 | 96,0 | 0,95 | >80±5 |
| CS 2 (n=8) | Porcine | 24 405 | 2,3 | 95,1 | 0,94 | >80±5 |
| CS 3 (n=3) | Porcine | 23 550 | 2,4 | 95,2 | 0,95 | 83±5 |
| CS 4 (n=3) | Bovine | 22000 | 1,7 | 95,9 | 0,93 | 70±5 |
| CS 5 (n=3) | Requin | 38 124 | 1,4 | 95,3 | 1,17 | 72±6 |

**Effet de CS de différentes sources sur les PG néosynthétisés par les chondroïtines dont la dégradation est induite par IL-1.**

## Revendications

1. Utilisation des chondroïtines sulfatées en 2, en 4 en 6 et/ou en 2,6 pour la production d'un médicament en tant que facteur de conservation ou de régénération dans les cartilages.

2. Utilisation selon la revendication 1, dans laquelle le médicament a pour principe actif un produit contenant essentiellement de l'acide chondroïtine 2-sulfurique.

3. Utilisation selon la revendication 1, dans laquelle le médicament a pour principe actif un produit contenant essentiellement de l'acide chondroïtine 6-sulfurique.

4. Utilisation selon la revendication 1, dans laquelle le médicament a pour principe actif un produit contenant essentiellement de l'acide chondroïtine 4-sulfurique.

5. Utilisation selon la revendication 1, dans laquelle le médicament a pour principe actif un produit contenant essentiellement de l'acide chondroïtine 2,6-disulfurique.

6. Utilisation selon la revendication 1, dans laquelle le médicament a pour principe actif une chondroïtine sulfurique.

7. Utilisation selon l'une des revendications 1 à 6, dans laquelle le médicament est un mélange de chondroïtines sulfuriques d'origine bovine, d'origine porcine ou d'origine ichtyque.

8. Utilisation selon l'une des revendications 1 à 7, dans laquelle le mélange d'acides chondroïtine sulfuriques est accompagné de molécules non sulfatées.

9. Utilisation selon l'une des revendications 1 à 8, dans laquelle le médicament est présenté sous forme de compositions pharmaceutiques destinées à la voie orale.

10. Utilisation selon l'une des revendications 1 à 8, dans laquelle le médicament est présenté sous forme de compositions pharmaceutiques destinées à la voie parentérale.

11. Utilisation selon l'une des revendications 1 à 8, dans laquelle la dose d'acide(s) chondroïtine sulfurique(s) par prise unitaire s'échelonne de 0,2 g à 2 g.
